# EUROPEAN PATENT SPECIFICATION

(11) **EP 4 229 215 B1**
(45) Date of publication and mention of the grant of the patent: **20.11.2024**
(21) Application number: 21798961.5
(22) Date of filing: 15.10.2021
(51) Int. Cl.: C12Q 1/6806

(54) **METHODS AND KITS FOR NUCLEIC ACID PURIFICATION**
VERFAHREN UND KITS ZUR NUKLEINSÄUREREINIGUNG
PROCÉDÉS ET KITS POUR LA PURIFICATION D'ACIDES NUCLÉIQUES

(30) Priority: 16.10.2020 CH 13382020
(43) Date of publication of application: 23.08.2023
(73) Proprietor: ProtonDX Limited, London W12 0BZ (GB)
(72) Inventor: GRASS, Robert N., 8046 Zürich (CH)
(74) Representative: Hamer, Thomas Daniel
(86) International application number: PCT/EP2021/078603
(87) International publication number: WO 2022/079236

(56) References cited:
- EP-A1- 1 201 752
- EP-A1- 1 932 913
- US-A1- 2008 187 905
- LAMBERT K J: "PRETREATMENT OF CELLS OF KLEBSIELLA PNEUMONIAE WITH 50% (V/V) DIMETHYLSULFOXIDE YIELDS PURIFIED DEOXYRIBONUCLEIC ACID OF LOW POLYSACCHARIDE CONTENT", AGRICULTURAL AND BIOLOGICAL CHEMISTRY, AGRICULTURAL CHEMICAL SOCIETY OF JAPAN, JP, vol. 46, no. 12, 1 January 1982 (1982-01-01), XP009031027, ISSN: 0002-1369

## Description

### FIELD OF THE INVENTION

The present invention relates to methods for purifying nucleic acids from biological samples using a solid phase material having a glass surface, and kits for purifying nucleic acids from a biological sample.

### BACKGROUND OF THE INVENTION

The various methods (e.g. PCR, RT-PCR, sequencing, NGS) employed in the biochemical and biomedical analysis of nucleic acids (e.g. RNA and DNA) from biological samples very often requires the purification of the nucleic acids from other sample constituents (e.g. cells, proteins, chemicals, etc.) prior to detection and analysis. To ensure that samples (e.g. from cells, bacteria, blood, sputum, swabs, soil, water) are compatible with these methods, the nucleic acids often have to first be purified, so that other components of the samples (e.g. nucleases, RNAses, DNAses, humic acids, solvents) do not interfere with the analytic procedure. Such nucleic acid purification steps are often also required in between various biochemical procedures (e.g. post PCR, pre PCR, pre RT-PCR, pre ligation, post gel-electrophoresis).

Following one or more preparative steps (e.g. digestion, lysis, homogenization, cell centrifugation, addition of storage solutions, addition of surfactants, stabilizers, denaturing agents, oxidation agents enzymes), the obtained biological sample containing the nucleic acids is subjected to purification. A commonly used technology to purify the nucleic acids from a sample involves firstly binding *via* adsorption the nucleic acids onto a glass surface, such as a silica glass surface or silica containing glass surface, of a solid phase material in presence of a binding buffer, which is an aqueous buffer comprising a chaotropic salt (e.g. guanidinium thiocyanate, guanidinium hydrochloride, urea) at high concentration (usually >1 M). In one of the commonly applied purification methods, the solid phase material having a glass surface is a fiber or beads column, such as silica fiber column, or silica beads column, through which the buffer solutions are passed using centrifugal or pressure drop induced forces. In an alternative purification method, the glass surfaces are supported on magnetic particles, where very different particle sizes can be used, ranging from ca. 20 nm to 10 micrometers. When using magnetic particles magnetic forces are used to separate the magnetic particles from the liquid. Magnetic particles based systems are easier to automate than the column based systems, and often find application when many samples have to be treated simultaneously, and/or when no centrifuge is available. Following binding of the nucleic acids to the glass surface of the solid-phase material, a second step of the purification method involves removal of the remaining contaminants (e.g. cells, proteins, chemicals, etc.) present in the biological sample and of the chaotropic salts by washing the glass surfaces carrying the nucleic acids with a wash buffer At least one washing step is required. Usually, the wash buffer used in the second step of the purification method is a solution containing at least 50-wt%, more commonly at least 70-wt% of a short chain aliphatic alcohol, such as ethanol or isopropanol. The remainder of the wash buffer is an aqueous buffer composition or deionized water. Solutions of polyethylene glycol may be alternatively used as wash buffers. The purification methods may differ in the precise composition of the wash buffer, and in the number of washing steps (usually 1-3 washing steps, either with the same wash buffer, or with wash buffers having different compositions). In a third step, the solid-phase material having glass surfaces carrying the nucleic acids are immersed in and/or flowed through by an elution buffer, optionally containing a low salt concentration (typically up to ca. 10 mM total salts) in which the nucleic acids are soluble so that the nucleic acids are desorbed from the glass surface of the solid-phase material. Generally, the elution buffer is deionized water, or TE buffer (i.e. a mixture of TRIS (tris(hydroxymethyl)aminomethane) buffer and EDTA (ethylenediaminetetraacetic acid)).

Prior to elution of the nucleic acids from the glass surface of the solid-phase material, it is important that the glass surfaces are largely free of wash buffer constituents, as these interfere with nucleic acid detection and analysis. Especially short chain alcohols, such as ethanol and isopropanol, are known to interfere with commonly employed enzymes, such as reverse transcriptases, polymerases, ligases etc. making the removal of the wash buffer constituents important. This phenomenon is less problematic when using the column based approach, as the columns can be dried effectively by centrifugation, or application of a pressure drop (positive pressure from one side and/or vacuum from the other side). However, even when the purification method relies upon the column based approach, ethanol contamination of the final sample can be a problem (see e.g. Jue et al. Scientific Reports 10, 1940 (2020)). For the purification methods using magnetic particles, the complete removal of the wash buffer constituents from the particles surface is even more difficult. When pelleting the particles using magnetic forces (usually using an external solid state magnet), the void spaces in-between the individual magnetic particles remain filled with wash buffer, even if the supernatant solution is removed by pipetting. This situation is usually worsened by the usage of small size magnetic particles (ca 50 nm to 1000 nm) as these tend not to pack as well as the larger size magnetic particles, resulting in more void spaces. To fully remove the volatile wash buffer constituents (usually mainly ethanol), the bead pellet is often left to dry prior the nucleic acid elution step. An alternative mode of action is to separate the magnetic particles from the wash buffer solution using magnetic combs, which are introduced into the buffer, and bind the magnetic particles to the surface of the combs, which are then removed from the wash solutions and let to dry prior to the elution step. The above-summarized drying steps (either with particles on magnetic combs or particles pelleted in a vessel) can take up considerable amounts of time (10-30 minutes), which can make up more than 50% of the whole nucleic acid purification process time, and render lengthy the purification method. Ways to shorten this drying time can be applied temperature, ventilation and larger elution volumes (effect of ethanol dilution in final nucleic acid solution), but none of these is ideal as they introduce complexity, risk of sample contamination and loss of sensitivity. The longer drying time of small size magnetic particles (more voids when compacted) has resulted in a widespread use of larger size magnetic particles (> 1000 nm), although these larger size particles have a distinct physical disadvantage, namely lower surface to volume ratio. The problem of voids is even enhanced, when aggregates of small size magnetic particles are utilized. Such aggregates are preferred in terms of offering a good combination of surface to volume ratio (increased binding capacity), and a high magnetic moment per particle (increased magnetic separation speed), but suffer from poor particle packing during compaction to a pellet and a corresponding large void fraction. Consequently, the problem of wash buffer drying is increased if glass coated, aggregated, magnetic particles of small primary particle size (< 200 nm) are utilized as solid-phase material having a glass surface i.e. as a nucleic acid carrier. As many reverse transcriptases are very susceptible to alcoholic inhibitors, the presence of a short chain alcohol in the samples is more problematic for the samples intended for reverse transcription polymerase chain reaction (RT-PCR) analysis of RNA than for the samples intended for the polymerase chain reaction (PCR) analysis of DNA. Ethanol contamination of samples containing nucleic acids is also detrimental for the isothermal amplification procedures. To solve this problem, Jue et al. (Scientific Reports 10, 1940 (2020)) proposed the introduction of a two-phase wash step in the purification step. In this method, a wash buffer, which is not, or only poorly, miscible with water is used to wash away contaminants prior to the elution step. However, the introduction of an additional liquid phase introduces the additional down-stream difficulty of phase separation, which may require additional hardware (e.g. US20190100747).

US2008/187905 A1, EP 1 932 913 A1, and EP 1 201 752 A1 all disclose a method for the purification of a nucleic acid, for binding a nucleic acid to a solid surface or for the detection of a nucleic acid. The procedure entails the selective binding of nucleic acids to glass surfaces in chaotropic salt solutions and separating the nucleic acids from contaminants such as agarose, proteins or cell residue. The material with the bound DNA or RNA may then be washed at least once e.g. with a mixture of 70 volume parts ethanol with 30 volume parts water ("70% Ethanol") or by suitable wash solu- tions not containing alcohol.

Thus, a need remains for improved methods for purification of nucleic acids, in particular for purification methods that are more simple and time-efficient than the known purification methods, do not require additional hardware, and furnish purified nucleic acids free, or substantially free, of short chain alcohols (i.e. C₂-C₄ aliphatic alcohols), which are known to inhibit the enzymatic activity of the enzymes involved in down-stream processes, in particular in reverse transcription polymerase chain reaction (RT-PCR) and isothermal amplification.

### SUMMARY OF THE INVENTION

Accordingly, it is the object of the present invention to provide a method for purifying nucleic acids from a sample containing nucleic acids to be purified that is simple and time-efficient compared to the know purification steps and furnishes purified nucleic acids free, or substantially free, of short chain alcohols (i.e. C₂-C₄ aliphatic alcohols), wherein the method comprises the following steps conducted in the order **i)** - **iv):**
**i)** contacting the sample with a solid phase material having a glass surface in presence of a binding buffer containing a chaotropic salt to bind the nucleic acids to the glass surface and obtain a solid phase material having the nucleic acids bound to its glass surface;
**ii)** washing the solid phase material having the nucleic acids bound to its glass surface with a first wash buffer containing a C₂-C₄ aliphatic alcohol;
**iii)** washing the solid phase material having the nucleic acids bound to its glass surface with a final wash buffer comprising at least about 50wt-% dimethyl sulfoxide; and
**iv)** eluting the nucleic acids from the glass surface with an elution buffer to provide purified nucleic acids.

A further aspect according to the present invention is directed to a kit for purifying nucleic acids from a sample containing nucleic acids to be purified by using the purification method claimed and described herein.

Also claimed and described herein is the use of a buffer containing at least 50wt-%, preferably 70wt-%, more preferably at least 90wt-%, of dimethyl sulfoxide for purifying nucleic acids.

### DETAILED DESCRIPTION

### Definitions

The following definitions are to be used to interpret the meaning of the terms discussed in the description and recited in the claims.

As used herein, the article "a/an" indicates one as well as more than one, and does not necessarily limit its referent noun to the singular.

As used herein, the term "at least" is meant to define one or more than one, for example one or two or three.

As used herein, the term "about" means that the amount or value in question may be the specific value designated or some other value in its neighborhood. Generally, the term "about" denoting a certain value is intended to denote a range within ± 5% of the value. As one example, the phrase "about 100" denotes a range of 100 ± 5, i.e. the range from 95 to 105. Preferably, the range denoted by the term "about" denotes a range within ± 3% of the value, more preferably ± 1 %. Generally, when the term "about" is used, it can be expected that similar results or effects according to the invention can be obtained within a range of ±5% of the indicated value.

As used herein, the term "and/or" means that either all or only one of the elements of said group may be present. For example, "A and/or B" shall mean "only A, or only B, or both A and B". In the case of "only A", the term also covers the possibility that B is absent, i.e. "only A, but not B".

The term "comprising" as used herein is intended to be non-exclusive and open-ended. Thus, for instance a solution comprising a compound A may include other compounds besides A. However, the term "comprising" also covers, as a particular embodiment thereof, the more restrictive meanings of "consisting essentially of" and "consisting of", so that for instance "a solution comprising A, B and optionally C" may also (essentially) consist of A and B, or (essentially) consist of A, B and C.

Surprisingly it was found that a method for purifying nucleic acids from a sample containing nucleic acids to be purified, wherein the method comprises the following steps conducted in the order **i)** - **iv):**
**i)** contacting the sample with a solid phase material having a glass surface in presence of a binding buffer containing a chaotropic salt to bind the nucleic acids to the glass surface and obtain a solid phase material having the nucleic acids bound to its glass surface;
**ii)** washing the solid phase material having the nucleic acids bound to its glass surface with a first wash buffer containing a C₂-C₄ aliphatic alcohol;
**iii)** washing the solid phase material having the nucleic acids bound to its glass surface with a final wash buffer comprising at least about 50wt-% dimethyl sulfoxide; and
**iv)** eluting the nucleic acids from the glass surface with an elution buffer to provide purified nucleic acids provides in an expedient manner samples of purified nucleic acids that are free, or substantially free, of C₂-C₄ aliphatic alcohols. Such samples are extremely useful for down-stream processes, such as RT-PCR, qPCR, isothermal amplification and similar technologies used in modern nucleic acid analysis. As well known to the skilled person, the methods of purification of nucleic acids relying upon adsorption of nucleic acids on a solid support, followed by washing steps, and desorption of the nucleic acids from the solid support, aim at removing impurities, such as nucleases, RNAses, DNAses, humic acids, lipids and proteins, from the sample and at providing a sample consisting essentially of nucleic acids in the elution buffer.

To ensure the adsorption of the nucleic acids to the glass surface of the solid phase material, it is preferred that the chaotropic salt is present in the binding buffer in a concentration of at least 1 molar, and more preferred in a concentration exceeding 1.5 molar. The binding buffer may further contain an additional salt, an organic solvent, such as ethanol, isopropanol, and ethylene glycol, and/or a water-soluble polymer, such as polyethylene glycol. Examples of binding buffers include, but are not limited to, TRIS (tris(hydroxymethyl)aminomethane) buffer, and sodium citrate buffer. Examples of chaotropic salts include, but are not limited to, guanidinium thiocyanate, guanidinium hydrochloride and urea.

As used herein the term "sample containing nucleic acids to be purified" refers to a sample obtained by subjecting a biological sample (e.g. cells, bacteria, soil, stool, biopsies, preserved samples, blood, sputum etc.) to one or more preparative steps (e.g. digestion, lysis, homogenization, cell centrifugation, addition of storage solutions, addition of surfactants, stabilizers, denaturing agents, oxidation agents enzymes). It is within the common knowledge of the skilled person to select depending on the nature of the biological sample, the preparative steps required for the preparation of the sample containing nucleic acids to be purified.

At step i) of the purification method claimed and described herein, at least 1 wt-%, preferably at least 50 wt-%, more preferably at least 80 wt-%, and most preferably 100 wt-% of the nucleic acids present in the sample contacted with the solid phase material are adsorbed on the glass surface of the solid phase material.

As used herein the term "glass surface" refers to a surface made of a material containing at least 50 wt-% silica. Examples of materials containing at least 50 wt-% silica, include, but are not limited to, pure or essentially pure silica, and doped silica containing less than 50 wt-%, preferably less than 30 wt-%, dopants selected from boron cation B³⁺ (e.g. B₂O₃), aluminium cation Al³⁺ (e.g. Al₂O₃), calcium cation Ca²⁺ (e.g. CaO), sodium cation Na⁺ (e.g. Na₂O), potassium cation K⁺ (e.g. K₂O), ferric cation (e.g. Fe₂O₃), cobalt cation Co²⁺ (e.g. CoO), chlorine Cl⁻ (e.g. CaCl₂), fluorine F⁻ (e.g. CaF₂), carbonate (e.g. Ca₂CO₃), and phosphate (e.g. Ca₃(PO₄)₂). Preferably, the glass surface is made of amorphous (pure or doped) silica.

As well known to the skilled person, the wording "washing the solid phase material having the nucleic acids bound to its glass surface with a first/final wash buffer" implies contacting the glass surface carrying the nucleic acids with a first/final wash buffer and removal of said buffer together with the impurities, such as nucleases, RNAses, DNAses, humic acids, lipids and proteins, still present in the sample.

As used herein the term "C₂-C₄ aliphatic alcohol" refers to an alcohol having two to four carbon atoms and at least one hydroxyl group. The aliphatic alcohol may contain two or three hydroxyl groups. Examples of C₂-C₄ aliphatic alcohols include, but are not limited to, ethanol, *n-*propanol, iso-propanol, ethylene glycol and glycerin. The C₂-C₄ aliphatic alcohol is preferably selected from ethanol, and iso-propanol. More preferably, the C₂-C₄ aliphatic alcohol is ethanol. Preferably, the C₂-C₄ aliphatic alcohol is present in the first wash buffer in an amount of at least 50 wt-%, preferably at least 70 wt-%. The first wash buffer may be a solution of the C₂-C₄ aliphatic alcohol described herein and water, such as deionized water, containing at least 50 wt-%, preferably at least 70 wt-%, more preferably at least 80 wt-% of the C₂-C₄ aliphatic alcohol. Alternatively, the first wash buffer may consist essentially of a commercially available C₂-C₄ aliphatic alcohol, such as ethanol or isopropanol.

As used herein the term "final wash buffer" refers to a buffer that is used in the last washing step i.e. following this washing step, the nucleic acids are eluted from the glass surface. The final wash buffer described herein comprises at least 50 wt-% DMSO, preferably at least 70 wt-% DMSO, more preferably at least 90 wt-% DMSO, most preferably at least 99 wt-% DMSO, such as commercially available DMSO. The final wash buffer may contain a complexation agent, such as diethylenetriaminepentaacetic acid (DTPA), and ethylenediaminetetraacetic acid (EDTA), an inorganic salt, preferably selected from lithium bromide, lithium iodide, calcium nitrate, sodium iodide, sodium nitrate, and potassium iodide, and/or boric acid.

As well known to the skilled person and used herein, the term "elution buffer" refers to water or an aqueous solution containing a low salt concentration (typically up to ca. 10 mM total salts). As the nucleic acids are soluble in the elution buffer, the use of the elution buffer results in the release of the nucleic acids from the glass surface of the solid-phase material. The elution buffer is preferably selected from water, and TE buffer (i.e. a mixture of TRIS (tris(hydroxymethyl)aminomethane) buffer and EDTA (ethylenediaminetetraacetic acid)). In a preferred embodiment, the elution buffer consists essentially of water, such as deionized water.

At step iv) ofthe purification method claimed and described herein, at least 1 wt-%, preferably at least 50 wt-%, more preferably at least 80 wt-%, and most preferably 100 wt-% of the nucleic acids bound to the glass surface of the solid phase material are released/eluted from the glass surface of the solid phase material.

In a preferred embodiment, the solid phase material having a glass surface comprises glass coated magnetic particles, preferably having a particle size of between 20 nm to 10 micrometers. It was further found that the combination of the final wash buffer described herein with glass coated magnetic particles having a magnetic saturation of at least 50 emu/g, preferably of at least 70 emu/g decrease significantly the time of the purification method. Preferably, the glass coated magnetic particles are magnetic particles consisting of a shell and one or more magnetic cores, wherein the one or more magnetic cores consists of a ferromagmetic, ferrimagnetic or superparamagnetic material, preferably a ferromagnetic material, coated with one or more graphene layers, and said shell consists of a glass, preferably a silicate glass. Such particles have been described for example by the European patent application publication n° EP 2 224 268 A1. Preferably, the glass coated magnetic particles are primary magnetic particles (i.e. magnetic particles consisting of a shell and one magnetic core, wherein the magnetic cores consists of a ferromagmetic, ferrimagnetic or superparamagnetic material, preferably a ferromagnetic material, coated with one or more graphene layers, and said shell consists of a glass, preferably a silicate glass) and/or aggregates of said primary magnetic particles. The primary particles have preferably an average primary particle diameter lower than 200 nm, more preferably lower than 100 nm, and even more preferably of between 50 nm and 100 nm. Preferably the glass shell of the primary magnetic particles has a thickness lower than 50 nm. The core of the primary magnetic particles is preferably a ferromagnetic metallic core, and more preferably consists essentially of iron, cobalt, or alloys containing iron and/or cobalt. Preferably the coating made of one or more graphene layers has a thickness lower than 10 nm.

In an alternative embodiment, the solid phase material having a glass surface is a porous column bed of adsorbent having a glass surface, preferably a silica column bed, more preferably a silica fiber column bed, or a silica beads column bed, preferably fixed into a plastic tube. Suitable silica beads are high purity silica beads with porosity of 50 to 300 Å, preferably about 100 Å, and particle sizes from 5 to 100 µm, preferably from 10 to 20 µm.

Step ii) of the method or purification claimed and described herein may be conducted several times, typically up to three times. When repeated, step ii) may be conducted either with the same first wash buffer, or with first wash buffers of different composition.

In the purification method claimed and described herein step ii) is preferably conducted once. This is particularly advantageous because it further decreases the amount of time required for purifying the nucleic acids.

The purification method according to the present invention provides purified nucleic acids that are free, or substantially free, ofC₂-C₄ aliphatic alcohols and are particularly compatible for enzymatic down-stream detection and analysis technologies, especially for reverse transcription polymerase chain reaction, and/or isothermal amplification.

A further aspect according to the present invention is directed to a kit for purifying nucleic acids from a sample containing nucleic acids to be purified comprising:
**a-1)** the solid phase material having a glass surface described herein;
**b**-**1**) the binding buffer described herein;
**c-1**) an aqueous buffer, preferably water, for preparing the first wash buffer described herein;
**d-1**) the final wash buffer described herein; and optionally
**e-1**) the elution buffer described herein.

The buffers, and the solid phase material having a glass surface contained by the kit according to the present invention are typically stored and supplied in individual containers. As used herein, the term "container" encompasses any object suitable for carrying or storing a liquid material, such as a flask or a pouch. In a preferred embodiment, the solid phase material having a glass surface comprises glass coated magnetic particles as described herein stored either in a storage solution, such as water or an aqueous buffer, or in the binding buffer described herein.

A further aspect according to the present invention is directed to the use of a buffer containing at least 50 wt-% of dimethyl sulfoxide (DMSO), preferably at least 70 wt-% DMSO, more preferably at least 90 wt-% DMSO, most preferably at least 99 wt-% DMSO, such as commercially available DMSO for purifying nucleic acids. The buffer may contain a complexation agent, such as diethylenetriaminepentaacetic acid (DTPA), and ethylenediaminetetraacetic acid (EDTA), an inorganic salt, preferably selected from lithium bromide, lithium iodide, calcium nitrate, sodium iodide, sodium nitrate, and potassium iodide, and/or boric acid.

If the solid phase material contained by the kit claimed and described herein comprises the glass coated magnetic particles described herein, the kit may further contain a pipette comprising a pipette body, a pipette tip and a lever, characterized in that
- the lever is attachable to, or attached to, the pipette body, and
- the lever comprises a magnet, and
- the lever allows movement of the magnet in a first position close to the pipette tip and a second position remote from the pipette tip.

In the first position the distance between the magnet and the pipette tip may be lower than 20 mm, preferably lower than 5 mm, when measured axially from the bottom (tight end) of the pipette tip towards to top (wide end) of the pipette tip.

In the pipette described herein, the movement of the lever allows either placing the magnet in a first position close to the pipette tip (< 5mm, preferably < 1 mm, measured radially from the outer surface of the pipette tip), or in a second position remote from the pipette tip (> 10 mm, preferably > 30mm, measured radially from the outer surface of the pipette tip).

The lever contained by the pipette described herein may be removable attached to the pipette body, or may be permanently attached to the pipette body.

Further, the lever described herein may further contain a spacing element ensuring that the magnet cannot touch the pipette tip.

A further aspect according to the present invention is related to an automated system for purifying and / or analysing nucleic acids, the automated system being adapted to receive the kit claimed and described herein.

An additional aspect according to the present invention is directed to a method for operating the automated system described herein, the method comprising the steps of connecting the kit claimed and described herein to the system and performing steps i) to iv) of the purification method claimed and described herein, optionally followed by reverse transcription polymerase chain reaction, and/or isothermal amplification.

Also described herein is a computer program element characterized by being adapted, when in use on a general purpose computer, to cause the computer to perform the steps of the method for purifying nucleic acids claimed and described herein, as well as a computer readable medium on which the computer program element is stored.

The present invention is based on established nucleic acid purification technology, involving the binding of nucleic acids to a glass surface of a solid phase material, and washing away contaminants with a series of wash buffers prior to eluting the nucleic acids into an aqueous solution and provides a final wash buffer containing at least 50 wt-% of dimethyl sulfoxide, which enables removal of the residual of the C₂-C₄ aliphatic alcohol. As it can be anticipated that residual wash buffer would remain in the purified nucleic acids sample independently of the choice of the wash buffer, the wash buffer used in the last washing step should not interfere with the down-stream analysis of the nucleic acids, but still function as a wash buffer. Ideally, the wash buffer used in the last washing step should dissolve the constituents of previous processing steps without impacting the binding of the nucleic acids on the glass surface of the solid-phase material. Consequently, the wash buffer to be used in the final washing step of the purification method i.e. prior to the elution step should a) have a good miscibility with water and C₂-C₄ aliphatic alcohols, such as ethanol, b) not impact the binding of the nucleic acids to the glass surface i.e. the nucleic acids should remain on the glass surface of the solid-phase material, when said material is immersed in said wash buffer (i.e. nucleic acids should be poorly soluble in the wash buffer), and c) not interfere significantly with down-stream enzymatic action (especially common reverse transcriptases and polymerases for RT-PCR, qPCR, isothermal amplification and similar technologies used in modern nucleic acid analysis). A purely aqueous system does not seem to be a good candidate for a final wash buffer, as DNA is greatly soluble in water, and can only be rendered insoluble by the addition of very high amounts of salts (e.g. guanidinium hydrochloride, guanidinium thiocyanate, urea), and/or other water soluble polymers (e.g. polyethylene glycols, polyanions), as e.g. present in the binding buffer, which are known to interfere with the downstream enzymes. Subsequently, an appropriate buffer has to be nonaqueous, similar to the already employed ethanolic wash buffers. Although, it has been reported that DNA has a solubility in dimethyl sulfoxide solutions (Bonner and Klibanov, Biotechnol. Bioeng. 2000, 68, 339-344), surprisingly it was found that above specifications a)-c) are met by the organic solvent dimethyl sulfoxide (DMSO), which is therefore an ideal candidate for the basis of a final wash buffer to be used in a final wash step. While DMSO is known to interfere with the structure of nucleic acids and interfere strongly with the UV-VIS detection of nucleic acids, neither property is particularly relevant if down-stream detection and analysis is based on enzymatic reactions (such as RT-PCR, qPCR and consequently sequencing). Current specifications of wash buffer include rapid evaporation, as this results in a shorter drying time, and consequently short chain aliphatic alcohols (e.g. ethanol, isopropanol) are a good choice. DMSO, on the other hand is amongst the general purpose solvents with the lowest volatility (boiling point of 189°C vs. 78°C for ethanol), and would never be considered if speed of evaporation is taken as specification. It is surprising that DMSO can be used as a wash buffer, as it has been reported that short nucleic acids have a solubility in aqueous DMSO mixtures, even with very high DMSO contents (Bonner and Klibanov, Biotechnol. Bioeng. 2000, 68, 339-344). However, the experiments conducted by the inventors (for e.g.: the comparative experiments 1 and 2 and experiments 3 - 5 according to the present invention) show no significant loss of nucleic acids during the DMSO wash step. Without being bound by the theory, it is believed that this finding may be a result of a tertiary interaction between the nucleic acid, the glass surface and the DMSO solvent. Also, DMSO is known for having an influence on enzymatic reactions involving nucleic acids, however this influence is usually beneficial (promoting enzymatic action). As shown in the herein described experiments, DMSO can be used as a final wash buffer in the purification and enzymatic detection of nucleic acids without the need of a bead drying step prior to the elution of the nucleic acids from the glass surface of the solid-phase material. The experiments were performed for the analysis of RNA via RT-PCR, thereby displaying compatibility of the protocol with both down-stream enzymes, reverse transcriptase and polymerase. The experiments further show that a glass surface drying step is required if a state-of-the-art ethanolic wash buffer is used as the last drying step in conjunction with small size magnetic particles (20 nm-1000 nm). Consequently, the use of DMSO as final wash buffer in the final wash step of the purification method claimed and described herein results in a significant shortening of the nucleic acid purification/detection routine. The effectivity of DMSO as buffer for the final wash step can be further increased by adding appropriate components to the final wash buffer, which decrease the nucleic acid solubility in the buffer and protect the nucleic acids from degradation. Appropriate components include, but are not limited to complexation agents, such as DTPA, and EDTA, inorganic salts, such as lithium bromide, lithium iodide, calcium nitrate, sodium iodide, sodium nitrate, and potassium iodide, and boric acid.

The DMSO containing final wash buffer claimed and described herein is especially effective in purifying viral and phage RNA from buccal swabs and cell suspensions using very small size magnetic particles. However, its applicability is much broader, including a magnitude of biological sample types (cells, bacteria, soil, stool, biopsies, preserved samples, blood, sputum etc.) and a range of enzymatic down-stream detection and analysis technologies of RNA and DNA (e.g.: PCR, qPCR, LAMP, sequencing, NGS, nanopore sequencing etc.), and a range of methods of contacting samples and buffers with glass surfaces (column beds, large magnetic particles, submicrometer magnetic particles, nanometric magnetic particles).

The purification method claimed and described herein is implemented by performing the last glass surface wash step with a final wash buffer comprising at least 50 wt-% DMSO, preferably at least 70 wt-% DMSO, more preferably at least 90 wt-% DMSO, such as 100 wt-% DMSO. The final wash buffer may contain a complexation agent, such as diethylenetriaminepentaacetic acid (DTPA), and ethylenediaminetetraacetic acid (EDTA), an inorganic salt, preferably selected from lithium bromide, lithium iodide, calcium nitrate, sodium iodide, sodium nitrate, and potassium iodide, and/or boric acid. Preferably, the inorganic salt is lithium bromide.

Depending on the nature of the biological sample (cells, bacteria, soil, stool, biopsies, preserved samples, blood, sputum etc.), several preparative steps (e.g. digestion, lysis, homogenization, cell centrifugation, addition of storage solutions, addition of surfactants, stabilizers, denaturing agents, oxidation agents enzymes) well known to a skilled person may be required , to provide the sample containing.

For application of the inventive method for the purification of the nucleic acids with magnetic particles, the steps following the preparative steps are:
a) mixing of the prepared sample with a binding buffer and glass coated magnetic particles resulting in the binding of a fraction (typically 1% to 100%) of the nucleic acids originally present in sample to the surface of the particles. The binding buffer contains at least a chaotropic salt and may contain further salts, solvents (e.g. ethanol, isopropanol, ethylene glycol) and water-soluble polymers (e.g. polyethylene glycol).
b) collecting the particles by the aid of an external magnetic field (e.g. by application of a solid state magnet)
c) removing the liquid supernatant from the particles
d) adding a first wash buffer to the particle pellet
e) collecting the particles by the aid of an external magnetic field
f) removing the liquid supernatant from the particles
g) adding a solution of at least 50wt-% DMSO (preferably > 70wt-% DMSO, more preferably > 90wt-%DMSO) to the particles. This solution may also contain additional components. The solution and the particles may be mixed
h) collecting the particles by the aid of an external magnetic field, and removing the liquid supernatant from the particles
i) adding an elution buffer essentially consisting of water, or other low-salt aqueous solutions to the particles. The elution buffer and elution procedure (time, mixing and temperature) are chosen so that a fraction (1% to 100%) of the nucleic acids originally bound to the particle surface is eluted from the particle surface to the elution buffer.
j) collecting the particles by the aid of an external magnetic field
k) collecting the liquid supernatant for nucleic analysis/detection.

It was further found that the DMSO containing final wash buffer described herein worked best and resulted in the fastest nucleic acid purification if glass coated magnetic particles of high saturation magnetization were utilized, with a magnetic saturation of at least 50 emu/g, more preferably greater than 70 emu/g. An increased nucleic acid loading can additionally be achieved if using aggregated particles with small primary particle size. The primary particles have preferably an average primary particle diameter lower than 200 nm, more preferably, lower than 100 nm. An especially suitable example of such preferred highly magnetic particles have a silica glass coating, primary particle sizes of 50-100 nm, with ferromagnetic, metallic cores, each protected by a thin (< 10 nm) carbon coating. The cores essentially consist of iron, cobalt, or alloys containing iron and/or cobalt. Such cores are then either individually, or as aggregates, coated with a thin (< 50 nm) coating of silicate glass. Such ferromagnetic particles are referred to as silica coated carbon coated metallic magnetic particles.

For application of the inventive method for the purification of the nucleic acids in column mode, the steps following the preparative steps are:
a) mixing of the prepared sample with a binding buffer and loading the mixture to a column containing a porous column bed with a glass surface, resulting in the binding of a fraction (typically 1% to 100%) of the nucleic acids originally present in sample to the surface of the glass. The binding buffer contains at least one chaotropic salt and may contain other salts, solvents (e.g. ethanol, isopropanol, ethylene glycol) and water soluble polymers. (e.g. polyethylene glycol).
b) passing the sample through the glass coated column material by the aid of centrifugation or the application of a pressure drop
c) discarding the flow through
d) adding a first wash buffer to column
e) passing the wash buffer through the glass coated column material by the aid of centrifugation or the application of a pressure drop
f) discarding the flow through
g) adding a solution of at least 50wt% DMSO (preferably > 70wt% DMSO, most preferably > 90wt%DMSO) to the column. This solution may also contain additional components.
h) passing the wash buffer through the glass coated column material by the aid of centrifugation or the application of a pressure drop
f) discarding the flow through
i) adding an elution buffer essentially consisting of water, or other low-salt aqueous solutions to the column. The elution buffer is passed through the column by the aid of centrifugation or the application of a pressure drop. Thereby the elution procedure (e.g. flow through time, elution buffer volume) are chosen so that a fraction (1% to 100%) of the nucleic acids originally bound to the glass surface is eluted from the particle surface to the elution buffer.
j) collecting the flow-through for nucleic analysis/detection.
l) processing the collected supernatant by an enzymatic reaction.

According to a further aspect, the present invention provides a kit for the purification of nucleic acids of one of the types A-C

### Type A:

- Flask comprising glass coated magnetic particles in storage buffer
- Flask comprising binding buffer
- Flask comprising first wash buffer, being an aqueous solution, to which the kit user adds a short chain alcohol (e.g. ethanol) prior to using the kit.
- Flask comprising final wash buffer, being a solution comprising at least 50wt% DMSO, preferably 70wt% DMSO, most preferably > 90wt% DMSO
- (Optional): Flask comprising elution buffer.

### Type B:

- Flask comprising glass coated magnetic particles in binding buffer
- Flask comprising first wash buffer, being an aqueous solution, to which the kit user adds a short chain alcohol (e.g. ethanol) prior to using the kit.
- Flask comprising final wash buffer, being a solution comprising at least 50wt% DMSO, preferably 70wt% DMSO, most preferably > 90wt% DMSO
- (Optional): Flask comprising elution buffer.

### Type C:

- Flask comprising first wash buffer, being an aqueous solution, to which the kit user adds a short chain alcohol (e.g. ethanol) prior to using the kit.
- Flask comprising final wash buffer, being a solution comprising at least 50wt% DMSO, preferably 70wt% DMSO, most preferably > 90wt% DMSO
- (Optional): Flask comprising elution buffer.
- a number of plastic consumables (one per nucleic acid sample to be processed), each consisting of a plastic column with a porous column bed, which has a glass surface (can also essentially consist of glass)

For all types of kits, the user might be asked to add a kit component prior to use.

Details regarding the binding buffer, in particular suitable and preferred binding buffer components, as well as details regarding the binding matrix, the first wash buffer solution and the elution solution were specified above in conjunction with the method according to the present invention. It is referred to the above disclosure which also applies here.

### EXAMPLES

The present invention is now described in greater detail with respect to non-limiting examples.

### Comparative experiment 1: purification method using ethanol wash buffer and bead drying step

To a 200 µl sample of MS2 RNA (Roche product number 10165948001) diluted 10000 fold, were added 300 µl of a binding buffer (4M guanidinium thiocyanate, 0.1 mM TRIS, 0.02 mg/ml glycogen, pH =7.5), 20 µl of glass coated magnetic particles (50 mg/ml silica coated carbon coated cobalt with a primary particle size < 100 nm) and 400 µl of isopropanol. Following mixing for 30 seconds and 3 minutes storage, the magnetic particles were separated to the wall of the tube by the aid of an external magnet, and the liquid supernatant was removed by the aid of a pipette. Following addition of 500 µl of wash buffer (20wt% H₂O, 80wt% ethanol), the magnetic pellet was mixed with the wash buffer by the aid of a pipette. The magnetic particles were again collected at the wall of the tube by the aid of an external magnet, and the liquid supernatant was removed. Following addition of 500 µl of wash buffer (20wt% H₂O, 80wt% ethanol), the magnetic pellet was mixed with the wash buffer by the aid of a pipette. The magnetic particles were again collected at the wall of the tube by the aid of an external magnet, and the liquid supernatant was removed. The pellet was left in the tube to dry for 20 minutes prior to the addition of 40µl of water, and mixing of the pellet with the water by the aid of a pipette. Following collection of the magnetic particles to the sidewall of the tube, 5 µl of the supernatant were analyzed by RT-qPCR using appropriate primer and cycling conditions, yielding a cycle threshold of 22.8, as calculated by the commercial data analysis software (Roche).

### Comparative experiment 2: Purification method using ethanol wash buffer and without bead drying step

To a 200 µl sample of MS2 RNA (Roche product number 10165948001) diluted 10000 fold, were added 300 µl of a binding buffer (4M guanidinium thiocyanate, 0.1 mM TRIS, 0.02 mg/ml glycogen, pH =7.5), 20 µl of glass coated magnetic particles (50 mg/ml silica coated carbon coated cobalt with a primary particle size < 100 nm) and 400 µl of isopropanol. Following mixing for 30 seconds and 3 minutes storage, the magnetic particles were separated to the wall of the tube by the aid of an external magnet, and the liquid supernatant was removed by the aid of a pipette. Following addition of 500 µl of wash buffer (20wt% H₂O, 80wt% ethanol), the magnetic pellet was mixed with the wash buffer by the aid of a pipette. The magnetic particles were again collected at the wall of the tube by the aid of an external magnet, and the liquid supernatant was removed. Following addition of 500 µl of wash buffer (20wt% H₂O, 80wt% ethanol), the magnetic pellet was mixed with the wash buffer by the aid of a pipette. The magnetic particles were again collected at the wall ofthe tube by the aid of an external magnet, and the liquid supernatant was removed. Immediately after removal of the liquid supernatant, 40µl of water were added to the tube, and the pellet was mixed with the water by the aid of a pipette. Following collection of the magnetic particles to the sidewall of the tube, 5 µl of the supernatant were analyzed by RT-qPCR using appropriate primer and cycling conditions for 40 cycles on a Roche Lightcycler 96 machine. The applied data analysis software (Roche) was unable to calculate a cycle threshold as the amplification data did not result in the expected s-type amplification curve.

### Experiment 3 according to the present invention: DMSO final wash buffer instead of bead drying step

To a 200 µl sample of MS2 RNA (Roche product number 10165948001) diluted 10000 fold, were added 300 µl of a binding buffer (4M guanidinium thiocyanate, 0.1 mM TRIS, 0.02 mg/ml glycogen, pH =7.5), 20 µl of glass coated magnetic particles (50 mg/ml silica coated carbon coated cobalt with a primary particle size < 100 nm) and 400 µl of isopropanol. Following mixing for 30 seconds and 3 minutes storage, the magnetic particles were separated to the wall of the tube by the aid of an external magnet, and the liquid supernatant was removed by the aid of a pipette. Following addition of 500 µl of wash buffer (20wt% H₂O, 80wt% ethanol), the magnetic pellet was mixed with the wash buffer by the aid of a pipette. The magnetic particles were again collected at the wall of the tube by the aid of an external magnet, and the liquid supernatant was removed. Following addition of 500 µl of wash buffer (100% DMSO), the magnetic pellet was mixed with the wash buffer by the aid of a pipette. The magnetic particles were again collected at the wall of the tube by the aid of an external magnet, and the liquid supernatant was removed. Immediately after removal of the liquid supernatant, 40µl of water were added to the tube, and the pellet was mixed with the water by the aid of a pipette. Following collection of the magnetic particles to the sidewall of the tube, 5 µl of the supernatant were analyzed by RT-qPCR using appropriate primer and cycling conditions, yielding a cycle threshold of 22.6, as calculated by the commercial data analysis software (Roche).

### Experiment 4 according to the present invention: DMSO final wash buffer instead of bead drying step

A buccal swab of an individual is mixed with 500 µl of water. After vortexing the swab is removed, and 100 µl of the sample solution are mixed with 300 µl lysis buffer (2M guanidinium thiocyanate, 25 mM sodium citrate, 0.02 mg/ml glycogen, pH =6.5), 300 µl isopropanol and 20 µl magnetic particles (TurboBeads RNA capture, product No. 5002, 50 mg/ml silica coated carbon coated cobalt with a primary particle size < 100 nm) in an 2 ml tube. Using a 1 ml Eppendorf Research plus pipette, to which a lever carrying a solid state magnet has been attached to, and keeping the magnet away from the pipette tip (off position => 50mm radially from surface of tip), the mixture is aspirated from the tube. In a next step the magnet is moved towards the surface of the pipette tip (on position = ca. 1 mm radially from surface of tip) by operating the lever with the finger, and the liquid of the tube is discarded from the tip. During this operation > 90% (by weight) of the particles collected within the inner wall of pipette tip in the vicinity of the external magnet and most>90% of the liquid has left the tube. Next, the magnet is again moved away from the pipette tip (off position) by actuating the lever and a 500 µl of an ethanol solution is aspirated from a 2 ml tube. The magnet is again placed in the vicinity ofthe pipette tip (on position), and the liquid solution is discarded, leaving > 90% (by weight) of the magnetic particles collected within the inner wall of the pipette tip. This washing step is repeated, whereas in the repetition the wash solution is mixed with the particles by 5 operations of the pipette (magnet in off position) prior to aspirating the mixture (magnet in off position). With the magnet in on position, the liquid solution is discarded, leaving > 90% (by weight) of the magnetic particles collected within the inner wall of the pipette tip. Next, the magnet is again moved away from the pipette tip (off position) by actuating the lever and a 500 µl of a dimethyl sulfoxide (DMSO) solution is aspirated from a 2 ml tube. The magnet is again placed in the vicinity of the pipette tip (on position), and the liquid solution is discarded, leaving > 90% (by weight) of the magnetic particles collected within the inner wall of the pipette tip. In a next step, 40 µl of elution buffer (deionized water), which has been previously been prepared in an 2 ml tube is aspirated with the pipette and the magnet in the off position. By 7 operations of the pipette (aspiration/elution), the particles are mixed with the elution buffer in the tube prior to aspirating the mixture (magnet in off position for this operation). After placing the magnet in the on position, the liquid solution is eluted into a fresh tube, leaving > 95% (by weight) of the magnetic particles collected within the inner wall of the pipette tip. The collected liquid solution from the last step is analyzed via real time quantitative polymerase chain reaction (RT-PCR) with primers specific for the human RNase P gene and yielded a quantitative qPCR threshold cycle of 26.92 cycles.

### Example 5 according to the present invention: final wash buffer containing DMSO and an inorganic salt instead of bead drying step

To a 2 µl sample comprising DNA (50 bp DNA ladder) with an initial concentration 25 ng/µl were added 300 µ1 lysis buffer (2M guanidinium thiocyanate, 25 mM sodium citrate, 0.02 mg/ml glycogen, pH =6.5), 300 µl isopropanol and 20 µl magnetic particles (TurboBeads RNA capture, product No. 5002, 50 mg/ml silica coated carbon coated cobalt with a primary particle size < 100 nm). The sample was mixed with the aid of a pipette, and the magnetic particles were separated from the solution by the use of an external solid state magnet. The supernatant was discarded, the particles washed once with 400 µl of ethanol by first adding the ethanol, mixing the beads with the ethanol, separating the beads from the solution by the aid of and external magnetic solid state magnet, removing and discarding the supernatant. This wash was repeated a second time, but instead of ethanol, a wash solution comprising 30mg/ml LiBr in DMSO was used. Following the discarding of the 2nd wash solution, 40 µl of elution buffer (deionized water) was added to the beads, and the beads were mixed with the water by the aid of a pipette. The beads were removed from the solution by the aid of an external solid state magnet, and the concentration of DNA in the elution buffer was measured by the use of the qubit fluorometer (Thermo Fisher) and the Qubit dsDNA HS kit (Thermo Fisher) to be 0.8 ng/µl.

## Claims

1. A method for purifying nucleic acids from a sample containing nucleic acids to be purified, wherein the method comprises the following steps conducted in the order i) - iv):
i) contacting the sample with a solid phase material having a glass surface in presence of a binding buffer containing a chaotropic salt to bind the nucleic acids to the glass surface and obtain a solid phase material having the nucleic acids bound to its glass surface;
ii) washing the solid phase material having the nucleic acids bound to its glass surface with a first wash buffer containing a C2-C4 aliphatic alcohol;
iii) washing the solid phase material having the nucleic acids bound to its glass surface with a final wash buffer comprising at least about 50wt-% dimethyl sulfoxide; and after step iii)
iv) eluting the nucleic acids from the glass surface with an elution buffer to provide purified nucleic acids.

2. The method according to claim 1, wherein the chaotropic salt is present in the binding buffer in a concentration of at least 1 molar.

3. The method according to claim 1 or 2, wherein the solid phase material having a glass surface comprises glass coated magnetic particles.

4. The method according to claim 3, wherein the glass coated magnetic particles have a magnetic saturation of at least 50 emu/g, preferably of at least 70 emu/g.

5. The method according to claim 3 or 4, wherein the glass coated magnetic particles are magnetic particles consisting of a shell and one or more magnetic cores, wherein the one or more magnetic cores consists of a ferromagmetic, ferrimagnetic or superparamagnetic material, preferably a ferromagnetic material, coated with one or more graphene layers, and said shell consists of an oxidic glass, preferably a silicate glass.

6. The method according to claim 1 or 2, wherein the solid phase material having a glass surface is a porous column bed of an adsorbent having a glass surface, preferably a silica column bed, more preferably a silica fiber column bed, or a silica beads column bed, preferably fixed into a plastic tube.

7. The method according to any one of claims 1 to 6, wherein the C2-C4aliphatic alcohol is present in the first wash buffer in an amount of at least 50wt-%, preferably at least 70wt-%.

8. The method according to any one of claims 1 to 7, wherein the final wash buffer comprises at least about 70wt-%, preferably at least about 90wt-%, dimethyl sulfoxide.

9. The method according to any one of claims 1 to 8, wherein the final wash buffer further comprises a complexation agent.

10. The method according to any one of claims 1 to 9, wherein the final wash buffer further comprises an inorganic salt.

11. The method according to any one of claims 1 to 10, wherein the elution buffer consists essentially of deionized water.

12. The method according to any one of the claims 1 to 11, wherein the purified nucleic acids are used for reverse transcription polymerase chain reaction, and/or isothermal amplification.

13. A kit for purifying nucleic acids from a sample containing nucleic acids to be purified comprising **a-1)** the solid phase material having a glass surface recited by any one of claims 1, 3, 4, 5 and 6;
**b-1)** the binding buffer recited by claim 1 or 2;
c-1) an aqueous buffer for preparing the first wash buffer recited by claim 1 or 7;
**d-1)** the final wash buffer recited by any one of claims 1, 8, 9, or 10; and optionally
**e-1)** the elution buffer as recited by claim 1 or 11.

14. The kit according to claim 13, wherein the solid phase material having a glass surface comprises glass coated magnetic particles stored either in a storage solution, or in the binding buffer recited by claim 1 or 2.

15. Use of a buffer containing at least 50 wt-% of dimethyl sulfoxide for purifying nucleic acids bound to a glass surface of a solid phase material.

## Patentansprüche

1. Verfahren zur Reinigung von Nukleinsäuren aus einer zu reinigende Nukleinsäuren enthaltenden Probe, wobei das Verfahren die folgenden Schritte umfasst, ausgeführt in der Reihenfolge i) - iv):
i) Inkontaktbringen der Probe mit einem eine Glasoberfläche aufweisenden Festphasenmaterial in Gegenwart eines Bindungspuffers, der ein chaotropes Salz enthält, zum Binden der Nukleinsäuren an die Glasoberfläche und Erhalten eines Festphasenmaterials, an dessen Glasoberfläche die Nukleinsäuren gebunden sind;
ii) Waschen des Festphasenmaterials, an dessen Glasoberfläche die Nukleinsäuren gebunden sind, mit einem ersten Waschpuffer, enthaltend einen C2-C4-aliphatischen Alkohol;
iii) Waschen des Festphasenmaterials, an dessen Glasoberfläche die Nukleinsäuren gebunden sind, mit einem Schluss-Waschpuffer, umfassend mindestens etwa 50 Gew.-% Dimethylsulfoxid; und nach dem Schritt iii)
iv) Eluieren der Nukleinsäuren von der Glasoberfläche mit einem Elutionspuffer zum Bereitstellen gereinigter Nukleinsäuren.

2. Verfahren nach Anspruch 1, wobei das chaotrope Salz im Bindungspuffer bei einer Konzentration von mindestens 1 molar vorhanden ist.

3. Verfahren nach Anspruch 1 oder 2, wobei das eine Glasoberfläche aufweisende Festphasenmaterial Glas-beschichtete magnetische Partikel umfasst.

4. Verfahren nach Anspruch 3, wobei die Glas-beschichteten magnetischen Partikel eine magnetische Sättigung von mindestens 50 emu/g, vorzugsweise mindestens 70 emu/g, aufweisen.

5. Verfahren nach Anspruch 3 oder 4, wobei es sich bei den Glas-beschichteten magnetischen Partikeln um magnetische Partikel handelt, bestehend aus einer Hülle und einem oder mehreren magnetischen Kernen, wobei der eine oder die mehreren magnetischen Kerne aus einem ferromagnetischen, ferrimagnetischen oder superparamagnetischen Material, vorzugsweise einem ferromagnetischen Material, das mit einer oder mehreren Graphen-Schichten beschichtet ist, bestehen, und die Hülle aus einem oxidischen Glas, vorzugsweise einem Silikatglas, besteht.

6. Verfahren nach Anspruch 1 oder 2, wobei das eine Glasoberfläche aufweisende Festphasenmaterial ein poröses Säulenbett aus einem eine Glasoberfläche aufweisenden Adsorbens, vorzugsweise ein Silica-Säulenbett, stärker bevorzugt ein Silica-Faser-Säulenbett oder ein Silica-Kügelchen-Säulenbett, ist, das vorzugsweise in ein Kunststoffröhrchen hinein fixiert wurde.

7. Verfahren nach einem der Ansprüche 1 bis 6, wobei der C2-C4-aliphatische Alkohol im ersten Waschpuffer in einer Menge von mindestens 50 Gew.-%, vorzugsweise mindestens 70 Gew.-%, vorhanden ist.

8. Verfahren nach einem der Ansprüche 1 bis 7, wobei der Schluss-Waschpuffer mindestens etwa 70 Gew.-%, vorzugsweise mindestens etwa 90 Gew.-% Dimethylsulfoxid, umfasst.

9. Verfahren nach einem der Ansprüche 1 bis 8, wobei der Schluss-Waschpuffer ferner ein Komplexiermittel umfasst.

10. Verfahren nach einem der Ansprüche 1 bis 9, wobei der Schluss-Waschpuffer ferner ein anorganisches Salz umfasst.

11. Verfahren nach einem der Ansprüche 1 bis 10, wobei der Elutionspuffer im Wesentlichen aus entionisiertem Wasser besteht.

12. Verfahren nach einem der Ansprüche 1 bis 11, wobei die gereinigten Nukleinsäuren für Reverse-Transkriptase-Polymerasekettenreaktion und/oder isotherme Amplifikation verwendet werden.

13. Kit zur Reinigung von Nukleinsäuren aus einer zu reinigende Nukleinsäuren enthaltenden Probe, umfassend a-1) das eine Glasoberfläche aufweisende Festphasenmaterial, erwähnt in einem der Ansprüche 1, 3, 4, 5 und 6;
b-1) den Bindungsbuffer, erwähnt in Anspruch 1 oder 2;
c-1) einen wässrigen Puffer zum Herstellen des ersten Waschpuffers, erwähnt in Anspruch 1 oder 7;
d-1) den Schluss-Waschpuffer, erwähnt in einem der Ansprüche 1, 8, 9 oder 10; und optional
e-1) den Elutionspuffer, wie erwähnt in Anspruch 1 oder 11.

14. Kit nach Anspruch 13, wobei das eine Glasoberfläche aufweisende Festphasenmaterial Glas-beschichtete magnetische Partikel umfasst, die entweder in einer Speicherlösung oder im Bindungspuffer, der in Anspruch 1 oder 2 erwähnt ist, aufbewahrt werden.

15. Verwendung eines Puffers, enthaltend mindestens 50 Gew.-% Dimethylsulfoxid, zur Reinigung von Nukleinsäuren, die an eine Glasoberfläche eines Festphasenmaterials gebunden sind.

## Revendications

1. Procédé de purification d'acides nucléiques d'un échantillon contenant des acides nucléiques à purifier, dans lequel le procédé comprend les étapes suivantes effectuées dans l'ordre i) à iv) :
i) mise en contact de l'échantillon avec un matériau en phase solide ayant une surface en verre en présence d'un tampon de liaison contenant un sel chaotropique pour lier les acides nucléiques à la surface en verre et obtenir un matériau en phase solide ayant les acides nucléiques liés à sa surface en verre ;
ii) lavage du matériau en phase solide ayant les acides nucléiques liés à sa surface en verre avec un premier tampon de lavage contenant un alcool aliphatique en C2-C4 ;
iii) lavage du matériau en phase solide ayant les acides nucléiques liés à sa surface en verre avec un tampon de lavage final comprenant au moins environ 50 % en poids de diméthylsulfoxyde ; et après l'étape iii)
iv) élution des acides nucléiques de la surface en verre avec un tampon d'élution pour fournir des acides nucléiques purifiés.

2. Procédé selon la revendication 1, dans lequel le sel chaotropique est présent dans le tampon de liaison en une concentration d'au moins 1 molaire.

3. Procédé selon la revendication 1 ou 2, dans lequel le matériau en phase solide ayant une surface en verre comprend des particules magnétiques revêtues de verre.

4. Procédé selon la revendication 3, dans lequel les particules magnétiques revêtues de verre ont une saturation magnétique d'au moins 50 emu/g, de préférence d'au moins 70 emu/g.

5. Procédé selon la revendication 3 ou 4, dans lequel les particules magnétiques revêtues de verre sont des particules magnétiques constituées d'une enveloppe et d'un ou plusieurs noyaux magnétiques, dans lequel le ou les noyaux magnétiques sont constitués d'un matériau ferromagmétique, ferrimagnétique ou superparamagnétique, de préférence un matériau ferromagnétique, revêtu d'une ou plusieurs couches de graphène, et ladite enveloppe est constituée d'un verre oxydique, de préférence un verre de silicate.

6. Procédé selon la revendication 1 ou 2, dans lequel le matériau en phase solide ayant une surface en verre est un lit de colonne poreuse d'un adsorbant ayant une surface en verre, de préférence un lit de colonne de silice, plus préférablement un lit de colonne de fibres de silice, ou un lit de colonne de billes de silice, de préférence fixé dans un tube en matière plastique.

7. Procédé selon l'une quelconque des revendications 1 à 6, dans lequel l'alcool aliphatique en C2-C4 est présent dans le premier tampon de lavage en une quantité d'au moins 50 % en poids, de préférence au moins 70 % en poids.

8. Procédé selon l'une quelconque des revendications 1 à 7, dans lequel le tampon de lavage final comprend au moins environ 70 % en poids, de préférence au moins environ 90 % en poids, de diméthylsulfoxyde.

9. Procédé selon l'une quelconque des revendications 1 à 8, dans lequel le tampon de lavage final comprend en outre un agent de complexation.

10. Procédé selon l'une quelconque des revendications 1 à 9, dans lequel le tampon de lavage final comprend en outre un sel inorganique.

11. Procédé selon l'une quelconque des revendications 1 à 10, dans lequel le tampon d'élution est constitué essentiellement d'eau désionisée.

12. Procédé selon l'une quelconque des revendications 1 à 11, dans lequel les acides nucléiques purifiés sont utilisés pour une réaction en chaîne par polymérase par transcription inverse et/ou une amplification isotherme.

13. Kit pour la purification d'acides nucléiques d'un échantillon contenant des acides nucléiques à purifier, comprenant a-1) le matériau en phase solide ayant une surface en verre défini dans l'une quelconque des revendications 1, 3, 4, 5 et 6 ;
b-1) le tampon de liaison défini dans la revendication 1 ou 2 ;
c-1) un tampon aqueux pour la préparation du premier tampon de lavage défini dans la revendication 1 ou 7 ;
d-1) le tampon de lavage final défini dans l'une quelconque des revendications 1, 8, 9 ou 10 ; et éventuellement
e-1) le tampon d'élution défini dans la revendication 1 ou 11.

14. Kit selon la revendication 13, dans lequel le matériau en phase solide ayant une surface en verre comprend des particules magnétiques revêtues de verre stockées soit dans une solution de stockage, soit dans le tampon de liaison défini dans la revendication 1 ou 2.

15. Utilisation d'un tampon contenant au moins 50 % en poids de diméthylsulfoxyde pour la purification d'acides nucléiques liés à une surface en verre d'un matériau en phase solide.
